# EUROPÄISCHE PATENTSCHRIFT

(11) **EP 2 577 062 B1**
(45) Veröffentlichungstag und Bekanntmachung des Hinweises auf die Patenterteilung: **26.12.2018**
(21) Anmeldenummer: 11723220.7
(22) Anmeldetag: 27.05.2011
(51) Int. Cl.: F04B 1/00, F04B 27/00, F04B 39/12, F04B 53/16, A61M 1/00, F04B 1/20, F04B 27/08

(54) **KOLBENPUMPENVORRICHTUNG**
PISTON PUMP DEVICE
DISPOSITIF DE POMPAGE À PISTON

(30) Priorität: 03.06.2010 CH 886102010
(43) Veröffentlichungstag der Anmeldung: 10.04.2013
(73) Patentinhaber: Medela Holding AG, 6340 Baar (CH)
(72) Erfinder: EBERT, Manuel, CH-6005 Luzern (CH); IMBODEN, David, CH-6340 Baar (CH); RAMELLA, Ivo, CH-6030 Ebikon (CH)
(74) Vertreter: Clerc, Natalia
(86) Internationale Anmeldenummer: PCT/CH2011/000126
(87) Internationale Veröffentlichungsnummer: WO 2011/150529

(56) Entgegenhaltungen:
- EP-A1- 0 791 366
- US-A- 2 872 101
- US-A- 4 451 211
- US-A1- 2003 017 064

## Beschreibung

### TECHNISCHES GEBIET

Die vorliegende Erfindung betrifft eine Kolbenpumpenvorrichtung gemäss Oberbegriff des Patentanspruchs 1. Diese Pumpenvorrichtung eignet sich insbesondere als Saug- bzw. Vakuumpumpe, insbesondere im medizinaltechnischen Bereich. Bevorzugte Anwendungsgebiete sind Drainagepumpen zum Absaugen von Körperflüssigkeiten.

### STAND DER TECHNIK

Kolbenpumpen mit Pumpenzylinder und Pumpenkolben sind aus den verschiedensten Anwendungsbereichen hinlänglich bekannt. Die Anmelderin vertreibt beispielsweise seit Jahren eine Drainagepumpe mit dem Namen Dominant 35c/i, welche zwei doppeltwirkende, gegenläufige Kolben/Zylindersysteme aufweist.

Weitere Kolbenpumpen sind beispielsweise in WO 95/33924 und EP 0 791 366 beschrieben.

Die US 2003/0017064 offenbart eine Kolbenpumpenvorrichtung mit den Merkmalen des Oberbegriffs des Anspruchs 1.

Kolbenpumpen sind Kräften ausgesetzt, welche die Laufruhe beeinträchtigen. Es werden diverse Arten von Lagern, insbesondere Wälz- und Gleitlager zur Lagerung einzelner Teile der Pumpe verwendet. Diese Lager sind jedoch Verschleiss ausgesetzt. Beispielsweise werden Gleitlager- oder Rillenkugellager immer auf derselben Seite belastet. Dies führt zu Klopfgeräuschen. Infolgedessen müssen die Lager nach einer gewissen Zeit ausgewechselt werden. Herkömmliche Rillenkugellager benötigen zudem eine gewisse Rotationsgeschwindigkeit, um einen Schmierfilm aufzubauen. Ist die Schwenkgeschwindigkeit des Pumpenzylinders relativ klein, so ist die Schmierwirkung ungenügend. Dies ist insbesondere bei Saugpumpen mit einstellbarem bzw. regelbarem Luftfluss problematisch, da bei Reduktion des Luftflusses auch die Schwenkgeschwindigkeit des Zylinders abnimmt.

### DARSTELLUNG DER ERFINDUNG

Es ist eine Aufgabe der Erfindung, eine Kolbenpumpe, insbesondere für Absaugpumpen im medizinaltechnischen Bereich zu schaffen, welche möglichst verschleissarm ausgebildet ist und eine grosse Laufruhe aufweist.

Diese Aufgabe löst eine Kolbenpumpenvorrichtung mit den Merkmalen des Anspruchs 1.

Die erfindungsgemässe Kolbenpumpenvorrichtung weist eine Trägereinheit, einen Pumpenzylinder und einen im Pumpenzylinder angeordneten Pumpenkolben auf. Der Pumpenzylinder weist eine Längsmittelachse und einen senkrecht zur Längsmittelachse angeordneten Zylinderboden auf. Der Pumpenkolben ist relativ zu diesem Zylinderboden bewegbar und der Pumpenzylinder ist mit einer Befestigungseinheit an der Trägereinheit befestigt. Erfindungsgemäss weist diese Befestigungseinheit eine Blattfeder auf, welche eine Schwenkbewegung des Pumpenzylinders bzw. der Längsmittelachse des Pumpenzylinders relativ zur Trägereinheit ermöglicht. Die Befestigungsvorrichtung kann am Mantel des Pumpenzylinders befestigt sein. Vorzugsweise ist sie jedoch am Zylinderboden befestigt.

Blattfedern haben den Vorteil, dass sie bei geringer Auslenkung praktisch verschleissfrei sind. Da der Pumpenzylinder nur relativ kleine Schwenkbewegungen ausführt, ist ein kleiner, innerhalb des elastischen Bereichs der Feder liegender Auslenkungswinkel der Blattfeder gewährleistet.

Die erfindungsgemässe Kolbenpumpenvorrichtung weist nicht nur eine verschleissarme Lagerung auf, sondern senkt auch Herstellungskosten, da auf Wälz- und Gleitlager verzichtet werden kann. Des Weiteren vereinfacht die Reduktion der beweglichen Teile die Herstellung und auch die Wartung und ermöglicht eine kompakte Bauweise.

Diese Kolbenpumpenvorrichtung lässt sich insbesondere in einer Saugpumpe einsetzen. Dank der Verwendung von mindestens einer Blattfeder als Lager lässt sich der Luftfluss der Saugpumpe bis zum Stillstand der Pumpe regeln bzw. steuern.

Vorzugsweise bildet die Befestigungseinheit eine Blattfedereinspannung, wobei diese Blattfedereinspannung den Pumpenzylinder bezüglich der Trägereinheit verankert, wobei die Blattfedern eine Schwenkbewegung des Zylinders erlauben.

Vorzugsweise ist pro Zylinder eine einzige Blattfeder vorhanden. Vorzugsweise bildet diese oder mindestens eine der Blattfedern im unausgelenkten Zustand eine Ebene, in welcher eine geradlinige Verlängerung der genannten Längsmittelachse verläuft.

Die Blattfeder weist eine freie Länge und eine Breite auf, wobei sie vorzugsweise bezüglich ihrer freien Länge biegsam ausgebildet ist. Als freie Länge wird die Länge zwischen zwei einander gegenüberliegenden Einspannungen bzw. Blattfederlagerungen der Blattfeder verstanden. Das Verhältnis von freier Länge zu Breite ist vorzugsweise kleiner als 1. Eine derart kurze Blattfeder ermöglicht eine kompakte Bauweise der Kolbenpumpenvorrichtung. Die Dicke der Blattfeder beträgt vorzugsweise circa 0.5 mm, deren freie Länge circa 13 mm und deren Breite circa 25 mm.

Die Blattfeder kann auf unterschiedlichste Weise an der Trägereinheit und am Pumpenzylinder befestigt sein. In einer bevorzugten Ausführungsform ist hierfür ein erstes zylinderseitiges Befestigungselement vorhanden, welches mit dem Pumpenzylinder einstückig geformt, an ihm angeschweisst oder in ihm vergossen ist. Dadurch ist eine grosse Stabilität gewährleistet. Ist das zylinderseitige erste Befestigungselement zudem im Zylinderboden angeordnet, ist die Stabilität noch weiter erhöht.

In einer bevorzugten Ausführungsform weist der Zylinderboden einen im wesentlichen planen Bereich zum Verschluss des Pumpenzylinders und eine nach aussen vorstehende, einstückig an diesen planen Bereich angeformte Befestigungsaufnahme zur Aufnahme des zylinderseitigen ersten Befestigungselements auf.

Das zylinderseitige erste Befestigungselement kann unterschiedlich gestaltet sein und aus unterschiedlichen Materialien gefertigt sein. Vorzugsweise ist es jedoch blockförmig, insbesondere quaderförmig, ausgebildet und/oder aus Metall gefertigt.

In einer bevorzugten Ausführungsform weist die Befestigungseinheit eine zylinderseitige Einspannvorrichtung und eine trägerseitige Einspannvorrichtung auf. Die Blattfeder weist ein erstes und ein zweites Ende auf, wobei das erste Ende in der zylinderseitigen Einspannvorrichtung eingespannt ist und das zweite Ende in der trägerseitigen Einspannvorrichtung eingespannt ist. Die zylinderseitige und die trägerseitige Einspannvorrichtung weisen je zwei Befestigungselemente auf, zwischen welchen die Enden der Blattfeder angeordnet sind, wobei die zwei Elemente jeweils gegenseitig verschraubt sind. Auch diese Anordnung erhöht die Stabilität.

Wird die Feder an mindestens einem Ende, vorzugsweise an beiden Enden eingespannt, lassen sich Ermüdungsbrüche der Blattfeder vermeiden oder zumindest minimieren. Gute Resultate wurden mit blockförmigen Befestigungselementen, insbesondere aus Metall, erzielt. Vorzugsweise erfolgt die Einspannung mittels Schrauben, wobei die Verwendung eines Drehmomentschlüssels empfehlenswert ist, um wiederum das Risiko von Ermüdungsbrüchen und Materialbeschädigungen zu minimieren. Zudem ist gewährleistet, dass sich die Verbindung nicht infolge von Vibrationen löst bzw. verschiebt.

Vorzugsweise besteht der Zylinder aus PPA oder mit Kohlenfasern verstärktem PPA. Dies erhöht die Laufruhe und verhindert ein Quietschen. Der Kolben besteht vorzugsweise auch aus PPA. Der Dichtring des Kolbes besteht vorzugsweise aus PTFE mit Kohlenfasern und/oder Glasfasern und Mineralien verstärkt. Dies führt zu einer Schmierung durch den ersten Abrieb der Kolbendichtung gegenüber der aus den oben genannten Materialien bestehenden Zylinderwandung und erhöht wiederum die Laufruhe.

Die erfindungsgemässe Kolbenpumpenvorrichtung kann aus einer einzigen Zylinder/Kolbenkombination bestehen oder aus mehreren, insbesondere zwei, drei, vier oder fünf derartigen Kombinationen zusammengesetzt sein. Die erfindungsgemässe Anordnung eignet sich insbesondere für ein zueinander parallel ausgerichtetes, gegenläufiges Zylinder/Kolbenpaar mit zwei doppelt wirkenden Zylindern und zwei Kolben, wobei jeder Zylinder mit einer eigenen Befestigungseinheit mit einer eigenen Blattfeder an der Trägereinheit befestigt ist.

Ist eine erfindungsgemässe Saugpumpe mit einem derartigen Zylinder/Kolbenpaar mit einem einstufigen Getriebe zwischen Motor und Kurbelwelle versehen, so lassen sich die Herstellungskosten senken und die Wartung der Pumpe vereinfachen. Zudem lässt sie sich in kompakter und platzsparender Form verwirklichen. Diese Vorteile werden noch optimiert, wenn als Getriebe lediglich ein Zahnriemen zwischen Kurbelwelle und Motor verwendet wird. Der Zahnriemen erlaubt zudem einen möglichst geräuscharmen Betrieb der Pumpe.

Vorzugsweise wirken die beiden Kolbenstangen der Kolben unter 90° Versatz auf die Kurbelwelle.

Vorzugsweise lässt sich die Drehzahl des Motors kontinuierlich regeln. Um eine einfache Bedienung zu ermöglichen, ist jedoch eine Einstell- und Steuereinheit vorhanden, mittels welcher wahlweise mindestens eine oder zwei, vorzugsweise genau drei diskrete Drehzahlen des Motors einstellbar sind. Dies lässt sich sowohl bei kontinuierlich regelbaren Motoren wie auch anderen Arten von Motoren verwirklichen.

Die erfindungsgemässe Kolbenpumpenvorrichtung eignet sich insbesondere als Saugpumpe im medizinaltechnischen Bereich, insbesondere als Drainagepumpe zum Absaugen von Körperflüssigkeiten oder Körperfetten.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Figur 1: eine perspektivische Darstellung einer Saugpumpe gemäss einer ersten Ausführungsform der Erfindung;
- Figur 2: eine Seitenansicht der Saugpumpe gemäss Figur 1;
- Figur 3: einen Längschnitt durch eine Kolbenpumpenvorrichtung gemäss einer zweiten Ausführungsform der Erfindung;
- Figur 4: eine perspektivische Darstellung eines Zylinderbodens mit zylinderseitigem Befestigungsteil und Blattfeder gemäss der in Figur 1 dargestellten Saugpumpe;
- Figur 5: der Zylinderboden gemäss Figur 4 mit zylinderseitigem Befestigungsteil und Blattfeder in einer Explosionsdarstellung und
- Figur 6: einen Querschnitt durch den Zylinderboden gemäss Figur 4.

### BESCHREIBUNG BEVORZUGTER AUSFÜHRUNGSFORMEN

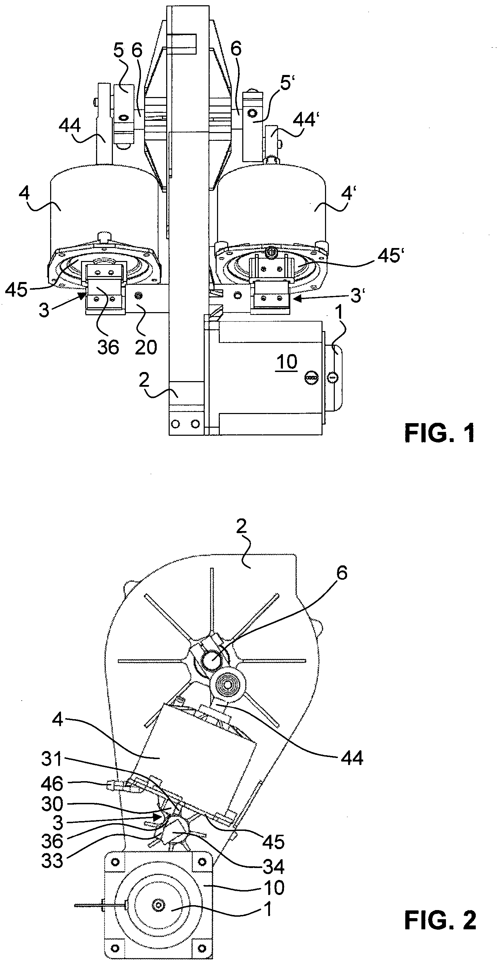

In den Figuren 1 und 2 ist eine Saugpumpe mit einer erfindungsgemässen Kolbenpumpenvorrichtung dargestellt. Sie weist einen Elektromotor 1 auf, welcher in einem Motorgehäuse 10 angeordnet ist. Mit dem Motorgehäuse 10 ist eine Trägereinheit 2 verbunden. Die Trägereinheit 2 umschliesst einen nicht dargestellten Zahnriemen, welcher einerseits mit einer Antriebswelle des Motors 1 verbunden ist und welcher andererseits eine Kurbelwelle 6 antreibt. Anstelle des Zahnriemens lassen sich auch andere ein- oder mehrstufige Getriebe einsetzen.

Die Kurbelwelle 6 ist an beiden Enden mit einer Kurbel 5, 5' verbunden. Jede Kurbel 5, 5' wirkt auf eine Kolbenstange 44, 44'. Die Kurbelwelle 6 wirkt dabei unter einem 90° Versatz auf die zwei Kolbenstangen 44, 44'. Die Kolbenstangen 44, 44' sind unter einem Versatz von 90° zueinander angetrieben.

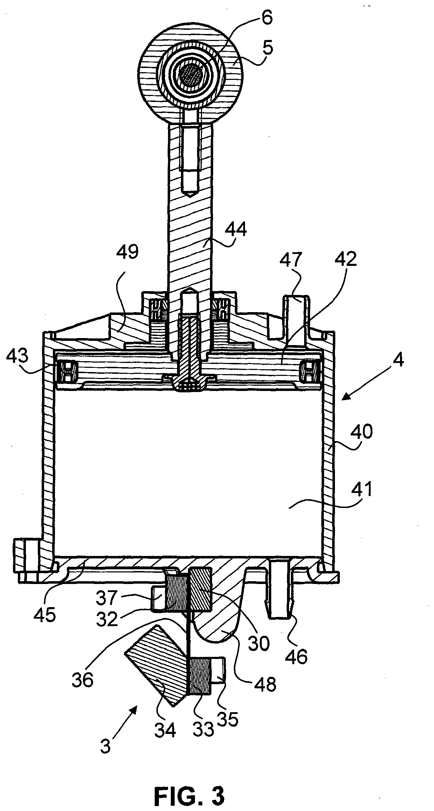

Die Kolbenstangen 44, 44' sind Teile einer Kolbenpumpenvorrichtung. Diese weist in diesem Beispiel zwei Pumpenzylinder 4, 4' und je einen im Pumpenzylinder 4, 4' bewegbar angeordneten Pumpenkolben 42 (siehe Figur 3).

In Figur 3 ist erkennbar, dass der Kolben 42 mit der Kolbenstange 44 verbunden ist und eine umlaufende Gleitdichtung 43 aufweist. Die Gleitdichtung 43 ist vorzugsweise aus PTFE gefertigt, wobei das Material vorzugsweise mit Kohlenfasern und/oder mit Glasfasern und Mineralien verstärkt ist. Der Zylinder besteht vorzugsweise aus PPA oder aus mit Kohlenfasern verstärktem PPA. Der Kolben besteht vorzugsweise aus PPA (Grivory), verstärkt mit Glasfasern und Mineralien. Typische Abmessungen des Zylinders 4 sind circa 75 mm für den Durchmesser und circa 58 mm für die Länge.

Der Zylinder 4, 4' weist eine Wandung 40, einen Hohlraum bzw. eine Pumpkammer 41 und einen Ein-/Auslass 46 im Zylinderboden 45, 45' sowie einen Ein-/Auslass 47 in einem dem Zylinderboden 45, 45' gegenüberliegenden Zylinderkopf 49 auf. Da es sich um eine doppeltwirkende Kolbenpumpenvorrichtung handelt, wirken die Ein-/Auslässe 46, 47 je nach Richtung der Bewegung der Kolbenstange als Einlass bzw. als Auslass. Die Ein-/Auslässe 46, 47 sind in bekannter Weise mit einem nicht dargestellten nach geschalteten Ventilblock verbunden.

Wie in den Figuren 1 und 2 ersichtlich ist, sind die Pumpenzylinder 4, 4' an der Trägereinheit 2 befestigt. Dieses weist hierfür einen quer zur ihrer Längsrichtung verlaufenden, auf beiden Seiten des Trägerhauptkörpers vorstehenden Trägerarm 20 auf. Die Trägereinheit 2 mit dem Trägerarm und das Motorgehäuse sind vorzugsweise aus Metall oder Kunststoff gefertigt.

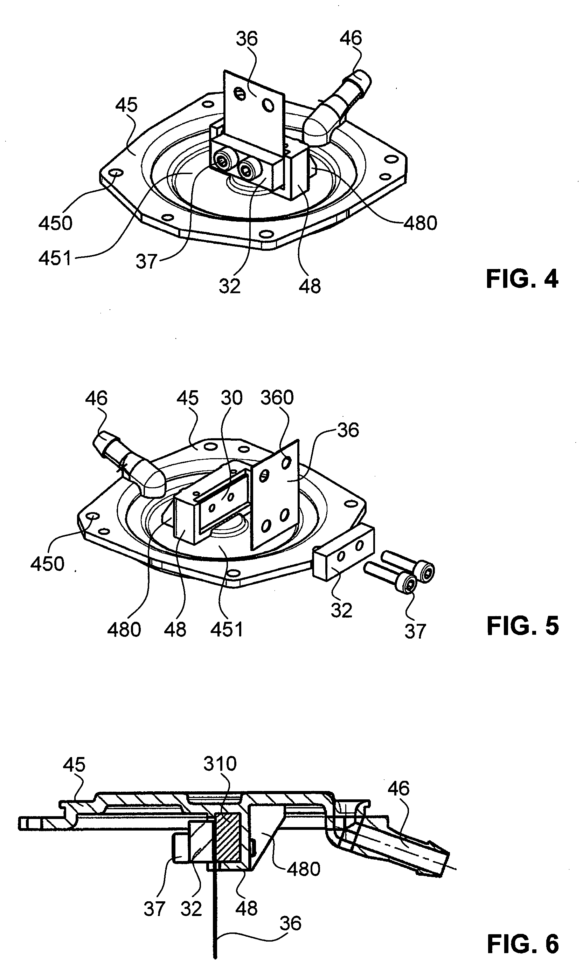

Die Pumpenzylinder 4, 4' sind über je eine Befestigungseinheit 3, 3' am Trägerarm 20 verankert. Vorzugsweise erfolgt diese Verankerung über einen Zylinderboden 45, 45'. Dieser Zylinderboden 45, 45' ist ortsfester Bestandteil des Pumpenzylinders 4, 4'. Der Kolben 42 bewegt sich relativ zu diesem Zylinderboden 45, 45'. Vorzugsweise ist er lösbar über eine Schraubverbindung mit dem restlichen Zylinder verbunden. Die entsprechenden Schraubenlöcher sind in den Figuren 4 und 5 mit der Bezugsziffer 450 gekennzeichnet. Der Zylinderboden 45, 45' ist vorzugsweise vertieft ausgebildet, insbesondere spritzgegossen, und weist Vertiefung 451 (Figur 5) und einen erhöhten umlaufenden Rand auf.

Erfindungsgemäss umfasst die Befestigungseinheit 3, 3' eine Blattfedereinspannung mit mindestens einer, vorzugsweise genau einer Blattfeder 36. Vorzugsweise sind keine anderen Lager, insbesondere Gleit-, Roll- oder Wälzlager zwischen Zylinder und Trägereinheit vorhanden.

Wie in Figur 3 erkennbar ist, weist die Befestigungseinheit 3, 3' eine zylinderseitige Einspannvorrichtung und eine trägerseitige Einspannvorrichtung auf. Beide Vorrichtungen bestehen je aus einem ersten und einem zweiten Befestigungselement 30, 32; 34, 33. Die Blattfeder 36 ist zwischen dem jeweiligen ersten Befestigungselement 30, 34 und dem zugehörigen zweiten Befestigungselement 32, 33 eingespannt. Hierzu sind vorzugsweise Schrauben 35, 37 vorhanden, welche das erste Befestigungselement 30, 34, die Befestigungslöcher 360 der Blattfeder 36 und das zweite Befestigungselement 32, 33 durchdringen. In den ersten Befestigungselementen 30, 34 und den zweiten Befestigungselementen 32, 33 sind hierzu entsprechende Löcher vorgesehen. Einige dieser Löcher sind in den Figuren 4 und 5 sichtbar.

Das zylinderseitige erste Befestigungselement 30 ist vorzugsweise fest im Zylinderboden 45 verankert. Der Zylinderboden 45 weist hierzu vorzugsweise eine dem übrigen Boden vorstehende Befestigungsaufnahme 48 auf. Vorzugsweise ist diese Aufnahme 48 einstückig mit dem restlichen Zylinderboden gefertigt, beispielsweise im Spritzgussverfahren. Die Aufnahme 48 überragt den Zylinder 4 auf dem dem Kolben 42 entgegen gesetzten Ende. Sie ist auf einer Seite verstärkt ausgebildet, beispielsweise durch eine sich zum Zylinderboden hin erweiternde Basis. Diese Verstärkung ist in den Figuren 4 bis 6 mit der Bezugsziffer 480 bezeichnet.

Die Aufnahme 48 weist eine Nut auf, in welcher das zylinderseitige erste Befestigungselement 30 befestigt wird. Vorzugsweise wird es eingegossen, eingeschweisst oder auf andere Weise fest mit der Aufnahme verbunden. Es ist somit vorzugsweise zumindest form- und stoffschlüssig mit dem Zylinder, genauer dem Zylinderboden, verbunden.

Wie in den Figuren 3 bis 6 erkennbar ist, wird die Blattfeder 36 mit ihrem zylinderseitigen Ende gemeinsam mit dem zylinderseitigen zweiten Befestigungselement 32 am zylinderseitigen ersten Befestigungselement 30 angeschraubt. Dasselbe geschieht mit dem zweiten trägerseitigen Ende der Blattfeder 36. Das trägerseitige erste Befestigungselement 34 und das zugehörige zweite Befestigungselement 33 sind in Figur 3 erkennbar. Auch das trägerseitige erste Befestigungselement 34 ist ähnlich bzw. gleich am Trägerarm 20 befestigt wie sein Gegenstück am Zylinderboden 45. Auch hier ist der Trägerarm 20 vorzugsweise mit einer verstärkten Aufnahme für eine form- und stoffschlüssige Verbindung mit dem trägerseitigen ersten Befestigungselement 34 versehen. Auch diese Aufnahme kann einstückig mit dem Trägerarm 20 hergestellt oder zumindest mit ihm verschweisst sein.

Die Ausführungsformen gemäss den Figuren 3 einerseits und den Figuren 4 bis 6 andererseits unterscheiden sich insbesondere in der Ausgestaltung des Zylinderbodens 45, insbesondere des Einlasses 46, und der Form der Aufnahme 48. Weitere Formen sind möglich.

Vorzugsweise ist zumindest das zylinderseitige erste Befestigungselement 30 aus Metall gefertigt. Vorzugsweise ist es block- oder quaderförmig ausgebildet. Vorzugsweise ist auch das trägerseitige erste Befestigungselement 34 in dieser Form und aus diesem Material ausgebildet. In einer bevorzugten Ausführungsform sind auch die zweiten Befestigungselemente 32, 33 derart ausgebildet.

Die Blattfeder 36 ist kurz ausgebildet. Ihre freie Länge zwischen den Einspannungen ist vorzugsweise kleiner als ihre Breite. Vorzugsweise ist die Länge zumindest nicht wesentlich grösser als die Breite. Typische Masse für freie Länge, Breite und Dicke sind circa 13 mm, 25 mm und 0.5 mm.

Wie in den Figuren 3 und 6 erkennbar ist, verläuft die Blattfeder 36 im wesentlichen in einer Ebene, in welcher auch die Längsmittelachse des Zylinders 4 verläuft. Im Betrieb kann nun der Zylinder 4, 4' dank der Blattfeder 36 eine Schwenkbewegung relativ zur Bewegungsrichtung des Kolbens 42 ausführen. Dies gilt für beide Zylinder 4, 4' gleichermassen, da die Befestigungsvorrichtung des zweiten Zylinders 4' mit derjenigen des ersten Zylinders 4 identisch ist.

Diese Anordnung lässt sich auch in anderen Kolbenpumpen verwirklichen. Die hier dargestellte Saugpumpe weist jedoch noch weitere vorteilhafte Merkmale auf, welche auch unabhängig von der oben beschriebenen erfindungsgemässen Befestigungsvorrichtung eingesetzt werden können. Der Motor 1 ist über eine hier nicht dargestellte Einstell- und Steuereinheit betätigbar. Diese Einheit weist insbesondere mindestens eine, vorzugsweise drei diskrete Einstellmöglichkeiten für den Betrieb des Motors auf. Dies sind beispielsweise drei Knöpfe oder ein Schalter mit drei Betriebstellungen. Aus Hygienegründen lassen sich hierfür auch kapazitive Sensoren, insbesondere mit drei Betriebsstellungen, verwenden. Der Schalter kann zudem noch eine Aus-Stellung aufweisen. Diese drei Betriebsstellungen wählen je eine diskrete Drehzahl des Motors und somit drei diskrete Saugleistungen der Pumpe, beispielsweise 40 l/min, 50 l/min und 60 l/min. Die mittlere Saugleistung entspricht dabei vorzugsweise einem Normalbetriebszustand bzw. einem empfohlenen Betriebszustand. Dadurch lässt sich die Bedienung der Pumpe vereinfachen, selbst wenn ein eigentlich stufenlos regelbarer Motor verwendet wird.

Die erfindungsgemässe Kolbenpumpenvorrichtung ist verschleiss- und geräuscharm ausgebildet. In Verwendung als Saugpumpe ermöglicht sie zudem einen regelbaren Luftfluss bis zum Stillstand der Pumpe.

**BEZUGSZEICHENLISTE**

| | | | |
|---|---|---|---|
| 1 | Elektromotor | 4 | erster Pumpenzylinder |
| 10 | Motorgehäuse | 4' | zweiter Pumpenzylinder |
| | | 40 | Zylinderwandung |
| 2 | Trägereinheit | 41 | Pumpkammer |
| 20 | Trägerarm | 42 | Pumpenkolben |
| | | 43 | Gleitdichtung |
| 3 | erste Befestigungseinheit | 44 | erste Kolbenstange |
| 3' | zweite Befestigungseinheit | 44' | zweite Kolbenstange |
| 30 | zylinderseitiges erstes Befestigungselement | 45 | erster Zylinderboden |
| | | 45' | zweiter Zylinderboden |
| 32 | zylinderseitiges zweites Befestigungselement | 450 | Befestigungsloch |
| | | 451 | Vertiefung |
| 33 | trägerseitiges erstes Befestigungselement | 46 | Einlass |
| | | 47 | Auslass |
| 34 | trägerseitiges zweites Befestigungselement | 48 | Befestigungsaufnahme |
| | | 480 | Verstärkung |
| 35 | Befestigungsschraube | 49 | Zylinderkopf |
| 36 | Blattfeder | | |
| 360 | Befestigungsloch | 5 | erste Kurbel |
| 37 | Befestigungsschraube | 5' | zweite Kurbel |
| | | 6 | Kurbelwelle |

## Patentansprüche

1. Kolbenpumpenvorrichtung mit einer Trägereinheit (2), einem Pumpenzylinder (4, 4') und einem im Pumpenzylinder (4, 4') angeordneten Pumpenkolben (42), wobei der Pumpenzylinder (4, 4') eine Längsmittelachse und einen senkrecht zur Längsmittelachse angeordneten Zylinderboden (45, 45') aufweist, wobei der Pumpenkolben (42) relativ zu diesem Zylinderboden (45, 45') bewegbar ist und wobei der Pumpenzylinder (4, 4') mit einer Befestigungseinheit (3) an der Trägereinheit (2) befestigt ist, wobei diese Befestigungseinheit (3) eine Blattfeder (36) aufweist, **dadurch gekennzeichnet, dass** die Blattfeder (36) derart angeordnet ist, dass sie eine Schwenkbewegung der Längsmittelachse des Pumpenzylinders (4, 4') relativ zur Trägereinheit (2) ermöglicht.

2. Kolbenpumpenvorrichtung nach Anspruch 1, wobei die Befestigungseinheit (3) am Zylinderboden (45, 45') befestigt ist.

3. Kolbenpumpenvorrichtung nach einem der Ansprüche 1 oder 2, wobei die Befestigungseinheit (3) eine Blattfedereinspannung bildet und wobei diese Blattfedereinspannung den Pumpenzylinder (4, 4') bezüglich der Trägereinheit (2) verankert.

4. Kolbenpumpenvorrichtung nach einem der Ansprüche 1 bis 3, wobei die Blattfeder (36) im unausgelenktem Zustand eine Ebene bildet und wobei eine geradlinige Verlängerung der genannten Längsmittelachse in dieser Ebene verläuft.

5. Kolbenpumpenvorrichtung nach einem der Ansprüche 1 bis 4, wobei die Blattfeder (36) eine freie Länge und eine Breite aufweist, wobei sie bezüglich ihrer freien Länge biegsam ausgebildet ist und wobei das Verhältnis von freier Länge zu Breite kleiner als 1 ist.

6. Kolbenpumpenvorrichtung nach einem der Ansprüche 1 bis 5, wobei die Befestigungseinheit (3) eine einzige Blattfeder (36) aufweist.

7. Kolbenpumpenvorrichtung nach einem der Ansprüche 1 bis 6, wobei ein zylinderseitiges erstes Befestigungselement (30) vorhanden ist, welches mit dem Pumpenzylinder (4, 4') einstückig geformt, an ihm angeschweisst oder in ihm vergossen ist.

8. Kolbenpumpenvorrichtung nach Anspruch 7, wobei das zylinderseitige erste Befestigungselement (30) im Zylinderboden (45, 45') angeordnet ist.

9. Kolbenpumpenvorrichtung nach Anspruch 8, wobei der Zylinderboden (45, 45') einen im Wesentlichen planen Bereich zum Verschluss des Pumpenzylinders (4, 4') und eine nach aussen vorstehende, einstückig an diesen planen Bereich angeformte Befestigungsaufnahme (48) zur Aufnahme des zylinderseitigen ersten Befestigungselements (30) aufweist.

10. Kolbenpumpenvorrichtung nach einem der Ansprüche 7 bis 9, wobei das zylinderseitige erste Befestigungselement (30) ein blockförmiges Metallstück ist.

11. Kolbenpumpenvorrichtung nach einem der Ansprüche 1 bis 10, wobei die Befestigungseinheit (3) eine zylinderseitige Einspannvorrichtung und eine trägerseitige Einspannvorrichtung aufweist, wobei die Blattfeder (36) ein erstes und ein zweites Ende aufweist und wobei das erste Ende in der zylinderseitigen Einspannvorrichtung eingespannt ist und das zweite Ende in der trägerseitigen Einspannvorrichtung eingespannt ist.

12. Kolbenpumpenvorrichtung nach Anspruch 11, wobei die zylinderseitige und die trägerseitige Einspannvorrichtung je ein erstes und ein zweites Befestigungselement (30, 32; 34, 33) aufweisen, wobei die Blattfeder (36) an ihren beiden Enden zwischen diesen Befestigungselementen (30, 32; 34, 33) eingespannt ist, wobei jeweils ein erstes und ein zweites dieser Befestigungselemente (30, 32; 34, 33) gegenseitig verschraubt sind .

13. Kolbenpumpenvorrichtung nach einem der Ansprüche 1 bis 12, wobei zwei Paare bestehend aus je einem Pumpenzylinder (4, 4'), je einem Kolben (42) und je einer Befestigungseinheit (3, 3') vorhanden sind, wobei die Längsmittelachsen der Pumpenzylinder (4, 4') im unausgelenkten Zustand der Pumpenzylinder (4, 4') parallel zueinander verlaufen.

14. Saugpumpe mit einer Kolbenpumpenvorrichtung nach einem der Ansprüche 1 bis 13, wobei die Saugpumpe ferner eine Kurbelwelle (6) zur Bewegung des Kolbens (42), einen Motor (1) zum Antrieb der Kurbelwelle (6) und ein Getriebe als Verbindung zwischen Motor (1) und Kurbelwelle (6) umfasst, wobei das Getriebe einstufig ausgebildet ist.

15. Saugpumpe nach Anspruch 14, wobei eine Einstell- und Steuereinheit vorhanden ist, mittels welcher wahlweise mindestens eine, vorzugsweise genau drei diskrete Drehzahlen des Motors (1) einstellbar sind.

## Claims

1. A piston pump device with a support unit (2), a pump cylinder (4, 4'), and a pump piston (42) arranged in the pump cylinder (4, 4'), the pump cylinder (4, 4') having a longitudinal center axis, and a cylinder bottom (45, 45') arranged perpendicular to the longitudinal center axis, the pump piston (42) being movable relative to this cylinder bottom (45, 45'), and the pump cylinder (4, 4') being secured on the support unit (2) by a securing unit (3), wherein this securing unit (3) has a leaf spring (36), **characterized in that** the leaf sprin g(36) is arranged such that it permits a pivoting movement of the longitudinal center axis of the pump cylinder (4, 4') relative to the support unit (2).

2. The piston pump device as claimed in claim 1, wherein the securing unit (3) is secured on the cylinder bottom (45, 45').

3. The piston pump device as claimed in either of claims 1 and 2, wherein the securing unit (3) forms a leaf spring clamp, and wherein this leaf spring clamp anchors the pump cylinder (4, 4') in relation to the support unit (2).

4. The piston pump device as claimed in one of claims 1 through 3, wherein the leaf spring (36) in the undeflected state forms a plane, and wherein a rectilinear continuation of said longitudinal center axis runs in this plane.

5. The piston pump device as claimed in one of claims 1 through 4, wherein the leaf spring (36) has a free length and a width, wherein the leaf spring (36) is made flexible in its free length, and wherein the ratio of free length to width is less than 1.

6. The piston pump device as claimed in one of claims 1 through 5, wherein the securing unit (3) has a single leaf spring (36).

7. The piston pump device as claimed in one of claims 1 through 6, wherein a cylinder-side first securing element (30) is present which is formed in one piece with the pump cylinder (4, 4') or is welded onto the latter or cast therein.

8. The piston pump device as claimed in claim 7, wherein the cylinder-side first securing element (30) is arranged in the cylinder bottom (45, 45').

9. The piston pump device as claimed in claim 8, wherein the cylinder bottom (45, 45') has a substantially plane area for closing the pump cylinder (4, 4'), and an outwardly protruding securing seat (48) which is formed integrally on this plane area and which serves to receive the cylinder-side first securing element (30).

10. The piston pump device as claimed in one of claims 7 through 9, wherein the cylinder-side first securing element (30) is a block-shaped metal piece.

11. The piston pump device as claimed in one of claims 1 through 10, wherein the securing unit (3) has a cylinder-side clamping device and a support-side clamping device, wherein the leaf spring (36) has a first end and a second end, and wherein the first end is clamped in the cylinder-side clamping device and the second end is clamped in the support-side clamping device.

12. The piston pump device as claimed in claim 11, wherein the cylinder-side clamping device and the support-side clamping device each have a first and a second securing element (30, 32; 34, 33), wherein the leaf spring (36) is clamped at its two ends between these securing elements (30, 32; 34, 33), and wherein a first and a second of these securing elements (30, 32; 34, 33) are in each case screwed together.

13. The piston pump device as claimed in one of claims 1 through 12, wherein two pairs consisting in each case of a pump cylinder (4, 4'), a piston (42) and a securing unit (3, 3') are present, and wherein the longitudinal center axes of the pump cylinders (4, 4') extend parallel to each other in the undeflected state of the pump cylinders (4, 4').

14. A suction pump with a piston pump device as claimed in one of claims 1 through 13, wherein the suction pump moreover comprises a crankshaft (6) for moving the piston (42), a motor (1) for driving the crankshaft (6), and a gear mechanism serving as connection between motor (1) and crankshaft (6), wherein the gear mechanism is a single-stage gear.

15. The suction pump as claimed in claim 14, wherein an adjustment and control unit is present, by means of which the motor (1) is adjustable to at least one speed, preferably exactly three discrete speeds.

## Revendications

1. Dispositif de pompage à piston comprenant une unité de support (2), un cylindre de pompe (4, 4') et un piston de pompe (42) disposé dans le cylindre de pompe (4, 4'), le cylindre de pompe (4, 4') présentant un axe médian longitudinal et un fond de cylindre (45, 45') disposé perpendiculairement à l'axe médian longitudinal, le piston de pompe (42) étant mobile par rapport à ce fond de cylindre (45, 45') et le cylindre de pompe (4, 4') étant fixé à l'unité de support (2) à l'aide d'une unité de fixation (3), cette unité de fixation (3) comprenant un ressort à lame (36), **caractérisé en ce que** le ressort à lame (36) est disposé de telle sorte qu'il permet un mouvement de pivotement de l'axe médian longitudinal du cylindre de pompe (4, 4') par rapport à l'unité de support (2).

2. Dispositif de pompage à piston selon la revendication 1, dans lequel l'unité de fixation (3) est fixée au fond de cylindre (45, 45').

3. Dispositif de pompage à piston selon l'une quelconque des revendications 1 et 2, dans lequel l'unité de fixation (3) forme un moyen de fixation de ressort à lame, et dans lequel ce moyen de fixation de ressort à lame ancre le cylindre de pompe (4, 4') par rapport à l'unité de support (2) .

4. Dispositif de pompage à piston selon l'une quelconque des revendications 1 à 3, dans lequel le ressort à lame (36) forme, à l'état non défléchi, un plan, et dans lequel un prolongement rectiligne dudit axe médian longitudinal s'étend dans ce plan.

5. Dispositif de pompage à piston selon l'une quelconque des revendications 1 à 4, dans lequel le ressort à lame (36) présente une longueur libre et une largeur, dans lequel le ressort est réalisé de manière souple par rapport à sa longueur libre, et dans lequel le rapport de la longueur libre à la largeur est inférieur à 1.

6. Dispositif de pompage à piston selon l'une quelconque des revendications 1 à 5, dans lequel l'unité de fixation (3) comprend un ressort à lame (36) unique.

7. Dispositif de pompage à piston selon l'une quelconque des revendications 1 à 6, dans lequel un premier élément de fixation (30) côté cylindre est prévu, lequel est formé d'une seule pièce avec le cylindre de pompe (4, 4'), est soudé sur celui-ci ou est coulé dans celui-ci.

8. Dispositif de pompage à piston selon la revendication 7, dans lequel le premier élément de fixation (30) côté cylindre est disposé dans le fond de cylindre (45, 45').

9. Dispositif de pompage à piston selon la revendication 8, dans lequel le fond de cylindre (45, 45') comprend une région sensiblement plane destinée à fermer le cylindre de pompe (4, 4') et un logement de fixation (48) faisant saillie vers l'extérieur et formé d'une seule pièce sur cette région plane, lequel le logement de fixation est destiné à loger le premier élément de fixation (30) côté cylindre.

10. Dispositif de pompage à piston selon l'une quelconque des revendications 7 à 9, dans lequel le premier élément de fixation (30) côté cylindre est une pièce métallique en forme de bloc.

11. Dispositif de pompage à piston selon l'une quelconque des revendications 1 à 10, dans lequel l'unité de fixation (3) comprend un dispositif de serrage côté cylindre et un dispositif de serrage côté support, le ressort à lame (36) comprenant une première extrémité et une deuxième extrémité, et la première extrémité étant serrée dans le dispositif de serrage côté cylindre et la deuxième extrémité étant serrée dans le dispositif de serrage côté support.

12. Dispositif de pompage à piston selon la revendication 11, dans lequel les dispositifs de serrage côté cylindre et côté support comprennent respectivement un premier et un deuxième élément de fixation (30, 32 ; 34, 33), le ressort à lame (36) étant, à ses deux extrémités, serré entre ces éléments de fixation (30, 32 ; 34, 33), un premier et un deuxième de ces éléments de fixation (30, 32 ; 34, 33) étant respectivement vissés mutuellement.

13. Dispositif de pompage à piston selon l'une quelconque des revendications 1 à 12, dans lequel deux paires constituées d'un cylindre de pompe (4, 4') respectif, d'un piston (42) respectif et d'une unité de fixation (3, 3') respective sont prévues, dans lequel les axes médians longitudinaux des cylindres de pompe (4, 4') s'étendent parallèlement l'un à l'autre à l'état non défléchi des cylindres de pompe (4, 4').

14. Pompe d'aspiration comprenant un dispositif de pompage à piston selon l'une quelconque des revendications 1 à 13, la pompe d'aspiration comportant en outre un vilebrequin (6) servant à déplacer le piston (42), un moteur (1) servant à entraîner le vilebrequin (6) et une transmission en tant que liaison entre le moteur (1) et le vilebrequin (6), la transmission étant à un étage.

15. Pompe d'aspiration selon la revendication 14, dans laquelle une unité de réglage et de commande est prévue, au moyen de laquelle sélectivement au moins un, de préférence exactement trois régimes distincts du moteur (1) peuvent être réglés.
